(19) **Europäisches Patentamt**

**European Patent Office**

**Office européen des brevets**

(11) Veröffentlichungsnummer: **0 428 137 B1**

(12) **EUROPÄISCHE PATENTSCHRIFT**

(45) Veröffentlichungstag der Patentschrift: **28.06.95**

(51) Int. Cl.6: **C12Q 1/00**, C12Q 1/54

(21) Anmeldenummer: **90121721.6**

(22) Anmeldetag: **13.11.90**

(54) **Verfahren zur Erhöhung der enzymatischen Reaktivität von Beta-Galactosidase.**

(30) Priorität: **14.11.89 DE 3937880**
**12.03.90 DE 4007836**

(43) Veröffentlichungstag der Anmeldung:
**22.05.91 Patentblatt 91/21**

(45) Bekanntmachung des Hinweises auf die
Patenterteilung:
**28.06.95 Patentblatt 95/26**

(84) Benannte Vertragsstaaten:
**AT BE CH DE DK ES FR GB GR IT LI LU NL SE**

(56) Entgegenhaltungen:
**EP-A- 0 049 475**

(73) Patentinhaber: **BOEHRINGER MANNHEIM GMBH**

**D-68298 Mannheim (DE)**

(72) Erfinder: **Herrmann, Rupert, Dr.
In der Au 23
W-8120 Weilheim (DE)**
Erfinder: **Guder, Hans-Joachim, Dr.
Schwattachweg 1
W-8120 Weilheim (DE)**
Erfinder: **Junius-Comer, Martina, Dr.
Am Bodenbach 5
W-8127 Iffeldorf (DE)**

(74) Vertreter: **Huber, Bernhard, Dipl.-Chem. et al
Patentanwälte
H. Weickmann, Dr. K. Fincke
F.A. Weickmann, B. Huber
Dr. H. Liska, Dr. J. Prechtel, Dr. B. Böhm
Postfach 86 08 20
D-81635 München (DE)**

Rank Xerox (UK) Business Services
(3.10/3.09/3.3.3)

**Beschreibung**

Die Erfindung betrifft ein Verfahren zur Erhöhung der enzymatischen Reaktivität von $\beta$-Galactosidase, sowie einen Testkit, der $\beta$-Galactosidase und ein von $\beta$-Galactosidase umsetzbares Substrat enthält.

$\beta$-Galactosidase ist ein gebräuchliches Marker-Enzym, das in immunologischen Testverfahren verwendet wird. Zur Durchführung solcher Testverfahren wird jedoch in den meisten Fällen eine beträchtliche Meßzeit benötigt, die z.B. bei schnell durchzuführenden Bestimmungen nach Unfällen und in medizinischen Notsituationen unter Umständen eine lebensbedrohliche Verzögerung des Beginns notwendiger therapeutischer Maßnahmen hervorrufen kann. Es besteht daher ein großer Bedarf, diese Reaktionszeiten zu verkürzen.

Darüberhinaus sind die für solche immunologischen Testverfahren benötigten Antikörper meistens nur mittels äußerst aufwendiger Verfahren herzustellen, weshalb ebenfalls versucht wird, die Antikörpermenge, die für einen solchen Test benötigt wird, so gering wie möglich zu halten. Da jedoch bei einer Verringerung der Antikörpermenge auch weniger Marker-Enzyme im Enzym-Immuno-Test vorliegen, nimmt dabei auch die Empfindlichkeit der Nachweisreaktion ab.

Andererseits ist es auch möglich, durch eine Erhöhung der Empfindlichkeit von solchen immunologischen Testverfahren eine geringere Menge an zu untersuchender Substanz einzusetzen. Dies spielt insbesondere dann eine Rolle, wenn nur eine begrenzte Menge an zu untersuchender Substanz zur Verfügung steht und damit eine Vielzahl von analytischen Untersuchungen durchgeführt werden soll.

Die Erfindung hat daher zum Ziel, die zuvor genannten Nachteile zu überwinden und Testverfahren mit einer erhöhten Empfindlichkeit bereitzustellen.

Dieses Ziel wird erfindungsgemäß durch ein Verfahren zur Erhöhung der enzymatischen Reaktivität von $\beta$-Galactosidase erreicht, welches dadurch gekennzeichnet ist, daß man dem Reaktionsgemisch ein Salz aus der Gruppe bestehend aus Azid, Rhodanid, Cyanat und Thiosulfat zusetzt. Es hat sich nämlich überraschenderweise gezeigt, daß die Zugabe solcher Salze zu einer Acceleration der enzymatischen Reaktivität von $\beta$-Galactosidase führt, was eine Erhöhung der Empfindlichkeit von solchen enzymatischen Testverfahren bedeutet, bei denen dieses Enzym verwendet wird. Die mit den zuvor genannten Acceleratorsalzen erzielte Steigerung der enzymatischen Reaktivität von $\beta$-Galactosidase-Reaktionen, insbesondere der hydrolytischen Spaltungs-Aktivität dieses Enzyms, ist besonders deswegen überraschend, weil andere Hydrolasen wie z.B. Glucosidase oder alkalische Phosphatase bei Einsatz gleicher Mengen (bezogen auf den $\beta$-Gal-Nachweis) dieser Acceleratorsalze nicht stimuliert, sondern sogar leicht inhibiert werden.

Von den Acceleratorsalzen sind die Thiosulfat-, Cyanat- und Rhodanidsalze bevorzugt, da sie mit den geringsten Mengen die erfindungsgemäße Wirkung erzielen. Der besonders geeignete Bereich liegt zwischen 0,5 und 10 mmol/l. Höhere Zusätze ergeben keine weitere Verbesserung. Bevorzugt werden 0,8 bis 5 mmol/l. Bei Azid liegt der geeignete Bereich zwischen 50 und 200 mmol/l. Auch hier sind höhere Konzentrationen noch wirksam, aber nicht besser. Bevorzugt werden 80 bis 150 mmol/l verwendet. Die Art des in diesen Salzen vorliegenden Kations ist zur Erzielung des erfindungsgemäßen Effektes ohne Bedeutung. Jedoch hat es sich als zweckmäßig erwiesen, die üblicherweise in biochemischen Reaktionen vorliegenden Kationen Lithium, Natrium, Kalium, Calcium, Magnesium und Ammonium zu verwenden.

Im erfindungsgemäßen Verfahren werden für die $\beta$-Galactosidase-Spaltung die üblichen dem Fachmann bekannten Substrate verwendet. Insbesondere werden zweckmäßigerweise solche Substrate verwendet, die eine photometrisch meßbare oder eine radioaktive Gruppe tragen. Es hat sich als vorteilhaft erwiesen, als Substrate Resorufin-$\beta$-D-galactopyranosid, Chlorphenolrot-$\beta$-D-galactopyranosid, 2-Nitrophenyl-$\beta$-D-galacto-pyranosid und/oder 2-Chlor-4-nitrophenyl-$\beta$-D-galactopyranosid sowie 2-Cyano-4-nitrophenyl-$\beta$-D-galactopyranosid und 2-Trifluormethyl-4-nitrophenyl-$\beta$-D-galactopyranosid (Herstellung gem. Deutsche Patentanmeldung P 40 21 063.4) zu verwenden.

Es hat sich überraschenderweise auch gezeigt, daß die erfindungsgemäße Erhöhung der enzymatischen Reaktivität von $\beta$-Galactosidase dann am stärksten ausgeprägt ist, wenn eine möglichst geringe Menge Enzym eingesetzt wird und dadurch das Substrat ohne Accelerator nur äußerst langsam gespalten wird. Schließlich hat es sich auch gezeigt, daß die Zugabe von Acceleratorsalz zu keiner nichtenzymatischen Hydrolyse des Substrates führt und daher der erfindungsgemäße Effekt nicht durch eine Hintergrundreaktion hervorgerufen wird oder von einer solchen überlagert ist.

Gegenstand der Erfindung ist auch ein Testkit, der als Marker-Enzym $\beta$-Galactosidase, ein von $\beta$-Galactosidase umsetzbares Substrat, sowie einen Accelerator in Form eines Azid-, Rhodanid-, Cyanat-und/oder Thiosulfatsalzes enthält.

Die Erfindung wird durch die folgenden Beispiele näher erläutert.

Beispiel 1

**Bestimmung von β-Galactosidase mit 2-Chlor-4-nitrophenyl-β-D-galactopyranosid (CNPG) als Substrat**

Reagenz:

0,1 mmol/l CNPG in Puffer I

Puffer I:

0,05 mol/l Kaliumphosphatpuffer, pH 7,0
0,16 mmol/l Magnesiumchlorid

Probe:

β-Galactosidase (spezifische Aktivität ca. 600 U/mg Protein bei 37°C mit 2-Nitrophenyl-β-D-galactosid als Substrat, EC 3.2.1.23,
Hersteller BOEHRINGER MANNHEIM GMBH, Best.-Nr. 745 731).
Die Durchführung der Bestimmung erfolgt in der Weise, daß Reagenz und Probe, ggf. unter Zugabe von Verstärker in Puffer I, zusammengegeben und der zeitliche Verlauf der Extinktion bei 405 nm bestimmt wird. Die Ergebnisse sind aus den Tabellen I, II, III, IIIa und IIIb zu ersehen.

## Tabelle I

## Ergebnisse für Kaliumrhodanid als Verstärker, Enzymkonzentration 0,3 µg/ml

**Messung 1: ohne Verstärker**
**Messung 2: mit 1 mmol/l Kaliumrhodanid**

3

| t (min) | Messung 1 | Messung 2 | Verstärkungsfaktor |
|---------|-----------|-----------|--------------------|
| 1 | 0,048 | 0,084 | 1,75 |
| 3 | 0,084 | 0,254 | 3,02 |
| 5 | 0,094 | 0,411 | 4,37 |
| 7 | 0,098 | 0,555 | 5,66 |
| 9 | 0,100 | 0,685 | 6,85 |
| 11 | 0,101 | 0,802 | 7,94 |
| 13 | 0,102 | 0,906 | 8,88 |

Tabelle II

Ergebnisse für Natriumazid als Verstärker, Enzymkonzentration
0,17 µg/ml

Messung 1:    ohne Verstärker

Messung 2:    mit 0,1 mol/l Natriumazid

| t [min] | Messung 1 | Messung 2 | Verstärkungsfaktor |
|---------|-----------|-----------|--------------------|
| 1 | 0,15 | 0,26 | 1,73 |
| 3 | 0,35 | 0,58 | 1,66 |
| 5 | 0,53 | 0,84 | 1,58 |
| 7 | 0,69 | 1,05 | 1,52 |
| 9 | 0,83 | 1,20 | 1,44 |
| 11 | 0,95 | 1,32 | 1,39 |
| 13 | 1,07 | 1,39 | 1,30 |

Tabelle III

Ergebnisse für Natriumazid als Verstärker, Enzymkonzentration 0,017 µg/ml

Messung 1: ohne Verstärker

Messung 2: mit 0,1 mol/l Natriumazid

| t [min] | Messung 1 | Messung 2 |
| --- | --- | --- |
| 0,5 | 0,0005 | 0,0035 |
| 3,5 | 0,001 | 0,009 |
| 5 | 0,001 | 0,011 |
| 7 | 0,001 | 0,015 |
| 9 | 0,001 | 0,019 |
| 11 | 0,001 | 0,023 |

Tabelle IIIa

Ergebnisse für Natriumthiosulfat als Verstärker, Enzymkonzentration 0,17 $\mu$g/ml

Messung 1: ohne Verstärker

Messung 2: mit 1 mmol/l Natriumthiosulfat

| t [min] | Messung 1 | Messung 2 |
| --- | --- | --- |
| 0,5 | 0,03 | 0,05 |
| 3 | 0,07 | 0,16 |
| 5 | 0,08 | 0,25 |
| 10 | 0,09 | 0,47 |
| 20 | 0,095 | 0,83 |

Tabelle IIIb

Ergebnisse für Kaliumcyanat als Verstärker, Enzymkonzentration: 0,17 $\mu$g/ml

Messung 1: ohne Verstärker

Messung 2: mit 1 mmol/l Kaliumcyanat

EP 0 428 137 B1

| t [min] | Messung 1 | Messung 2 |
|---|---|---|
| 1 | 0.040 | 0.045 |
| 2 | 0.060 | 0.075 |
| 3 | 0.070 | 0.100 |
| 4 | 0.075 | 0.115 |
| 5 | 0.080 | 0.130 |
| 6 | 0.085 | 0.150 |
| 10 | 0.090 | 0.190 |
| 15 | 0.095 | 0.240 |

Beispiel 2

**Bestimmung von $\beta$-Galactosidase mit Resorufin-$\beta$-Galactosid (RG) als Substrat**

Reagenz:

0,04 mmol/l RG in Puffer I
Die Durchführung der Bestimmung erfolgt wie in Beispiel 1 beschrieben.
Die Ergebnisse zeigen die Tabellen IV bis VI

**Tabelle IV**

**Ergebnisse für Kaliumrhodanid als Verstärker, Enzymkonzentration 0,17 µg/ml**

**Messung 1: ohne Verstärker**
**Messung 2: mit 1 mmol/l KSCN**

6

| t [min] | Messung 1 | Messung 2 |
|---------|-----------|-----------|
| 1 | 0,23 | 0,27 |
| 3 | 0,48 | 0,58 |
| 5 | 0,68 | 0,85 |
| 7 | 0,86 | 1,10 |
| 9 | 1,02 | 1,28 |
| 11 | 1,16 | 1,46 |
| 13 | 1,24 | 1,58 |

## Tabelle V

Ergebnisse für Kaliumrhodanid als Verstärker, Enzymkonzentration 0,017 µg/ml

Messung 1: ohne Verstärker
Messung 2: mit 1 mmol/l KSCN

| t [min] | Messung 1 | Messung 2 |
|---------|-----------|-----------|
| 1 | 0,012 | 0,018 |
| 3 | 0,030 | 0,045 |
| 5 | 0,045 | 0,074 |
| 7 | 0,055 | 0,102 |
| 9 | 0,065 | 0,132 |
| 11 | 0,070 | 0,160 |
| 13 | 0,074 | 0,190 |

## Tabelle VI

Ergebnisse für Natriumazid als Verstärker, Enzymkonzentration 0,17 µg/ml

Messung 1:     ohne Verstärker
Messung 2:     mit 0,1 mol/l $NaN_3$

| t [min] | Messung 1 | Messung 2 |
|---------|-----------|-----------|
| 1 | 0,08 | 0,14 |
| 3 | 0,18 | 0,33 |
| 5 | 0,25 | 0,50 |
| 7 | 0,32 | 0,66 |
| 9 | 0,36 | 0,80 |
| 11 | 0,40 | 0,93 |
| 12,5 | 0,42 | 1,02 |

Beispiel 3

**Bestimmung von $\beta$-Galactosidase mit Chlorphenolrot-$\beta$-Galacto-sid (CPG) als Substrat**

Reagenz:

0,1 mmol/l CPG in Puffer I

Die Durchführung der Bestimmung erfolgt wie in Beispiel 1 beschrieben. Die Ergebnisse zeigen die Tabellen VII, VIII und IX.

## Tabelle VII

Ergebnisse für Kaliumrhodanid als Verstärker, Enzymkonzentration 0,17 µg/ml

Messung 1: ohne Verstärker
Messung 2: mit 1 mmol/l KSCN

| t [min] | Messung 1 | Messung 2 |
|---------|-----------|-----------|
| 5 | 0,37 | 0,46 |
| 10 | 0,68 | 0,88 |
| 15 | 0,98 | 1,26 |

Tabelle VIII

Ergebnisse für Kaliumrhodanid als Verstärker, Enzymkonzentration 0,017 $\mu$g/ml

Messung 1:     ohne Verstärker
Messung 2:     mit 1 mmol/l KSCN

| t [min] | Messung 1 | Messung 2 |
|---------|-----------|-----------|
| 5 | 0,022 | 0,034 |
| 10 | 0,032 | 0,068 |
| 15 | 0,040 | 0,104 |
| 20 | 0,044 | 0,144 |
| 25 | 0,046 | 1,184 |

Tabelle IX

Ergebnisse für Natriumazid als Verstärker, Enzymkonzentration 0,17 $\mu$g/ml

Messung 1:     ohne Verstärker
Messung 2:     mit 0,1 mol/l NaN$_3$

| t [min] | Messung 1 | Messung 2 |
|---------|-----------|-----------|
| 5       | 0,26      | 0,63      |
| 10      | 0,50      | 1,18      |

**Patentansprüche**

1.  Verfahren zur Erhöhung der enzymatischen Reaktivität von $\beta$-Galactosidase, **dadurch gekennzeichnet,** daß man dem Reaktionsgemisch ein Salze aus der Gruppe bestehend aus Azid, Rhodanid, Cyanat und Thiosulfat zusetzt.

2.  Verfahren nach Anspruch 1, **dadurch gekennzeichnet**, daß das in den Salzen vorliegende Kation aus der Gruppe bestehend aus Lithium, Natrium, Kalium, Calcium, Magnesium und $NH_4^+$ gewählt wird.

3.  Verfahren nach einem der vorhergehenden Ansprüche, **dadurch gekennzeichnet,** daß man ein $\beta$ Galactosidase Substrat aus der Gruppe bestehend aus Resorufin-$\beta$-D-galactopyranosid, Chlorphenolrot-$\beta$-D-galactopyranosid, 2-Nitrophenyl-$\beta$-D-galactopyranosid und/oder 2-Chlor-4-nitrophenyl-$\beta$-D-galactopyranosid verwendet.

4.  Testkit, enthaltend $\beta$-Galactosidase, ein von $\beta$-Galactosidase umsetzbares Substrat und ein Salz aus der Gruppe bestehend aus Azid, Rhodanid, Cyanat und Thiosulfatsalz.

**Claims**

1.  Method for increasing the enzymatic reactivity of $\beta$-galactosidase, **wherein** a salt from the group comprising aside, thiocyanate, cyanate and thiosulphate is added to the reaction mixture.

2.  Method as claimed in claim 1, **wherein** the cation present in the salts is selected from the group comprising lithium, sodium, potassium, calcium, magnesium and $NH_4^+$.

3.  Method as claimed in one of the previous claims, **wherein** a substrate from the group comprising the $\beta$-galactosidase substrate resorufin-$\beta$-D-galactopyranoside, chlorophenol-red-$\beta$-D-galactopyranoside, 2-nitrophenyl-$\beta$-D-galactopyranoside and/or 2-chloro-4-nitrophenyl-$\beta$-D-galactopyranoside is used.

4.  Test kit containing $\beta$-galactosidase, a substrate which can be converted by $\beta$-galactosidase and a salt from the group comprising aside, thiocyanate, cyanate and thiosulphate salt.

**Revendications**

1.  Procédé pour augmenter la réactivité enzymatique de la $\beta$-galactosidase, caractérisé en ce que l'on ajoute au mélange réactionnel un sel du groupe consistant en les azotures, thiocyanates, cyanates et thiosulfates.

2.  Procédé selon la revendication 1, caractérisé en ce que le cation présent dans les sels est choisi dans le groupe consistant en le lithium, le sodium, le potassium, le calcium, le magnésium et $NH_4^+$.

3.  Procédé selon l'une des revendications précédentes, caractérisé en ce que l'on utilise un substrat du groupe consistant en le substrat de $\beta$-galactosidase résorufine-$\beta$-D-galactopyranoside, rouge de chlorophénol-$\beta$-D-galactopyranoside, 2-nitrophényl-$\beta$-D-galactopyranoside et/ou 2-chloro-4-nitrophényl-$\beta$-D-galactopyranoside.

4. Trousse de test contenant de la $\beta$-galactosidase, un substrat transformable par la $\beta$-galactosidase et un sel du groupe consistant en les azotures, thiocyanates, cyanates et thiosulfates.